# EUROPEAN PATENT APPLICATION

(11) **EP 2 923 693 A1**
(43) Date of publication of application: **30.09.2015**
(21) Application number: 13856722.7
(22) Date of filing: 25.11.2013
(51) Int. Cl.: A61K 8/98, A61K 35/64, A61Q 19/00

(54) **ANTI-AGING COSMETIC COMPOSITION COMPRISING FAT-SOLUBLE FRACTIONS OF BEE VENOM, AND METHOD FOR PREPARING SAME**

(30) Priority: 26.11.2012 KR 20120134433
(71) Applicant: Wissen Co., Ltd, Chungcheongnam-do 312-942 (KR)
(72) Inventor: AHN, Chang Ki, Daejeon 305-301 (KR); PARK, Jin Kyu, Daejeon 305-755 (KR); JEON, Jong Woon, Daejeon 305-755 (KR)
(74) Representative: Cabinet Chaillot
(86) International application number: PCT/KR2013/010714
(87) International publication number: WO 2014/081255

(57) **Abstract**

The present invention relates to a skin anti-aging cosmetic using bee venom. More specifically, the present invention relates to a cosmetic composition including lipid-soluble ingredients of bee venom. The cosmetic composition includes a lipid-soluble fraction and a water-soluble fraction obtained by fractionation of bee venom with a lipid-soluble solvent and a water-soluble solvent, respectively. The lipid-soluble fraction and the water-soluble fraction are in a volume ratio of 1:0.01-0.5. The cosmetic composition has anti-aging effects, such as reduction of skin wrinkles.

## Description

### Technical Field

The present invention relates to a skin anti-aging cosmetic using bee venom. More specifically, the present invention relates to a cosmetic composition including lipid-soluble ingredients of bee venom that has anti-aging effects, such as reduction of skin wrinkles, and methods for preparing the cosmetic composition.

### Background Art

Bee venom is a toxic liquid coming from the honeybee's ovipositor. Bee venom is a natural physiologically active substance of animal origin that is stored in the poison sac located at the tip of the bee's abdomen and connected to the sting and is secreted when the bee is stimulated. There are records that bee venom was used for therapy in ancient Egypt and Babylonia. Also in Korea, records that live bee stings were used to alleviate pain and treat rheumatoid arthritis were written in the Mawangdui medical texts (the first medical literature regarding acupuncture and moxibustion), Donguibogam (Principles and Practice of Eastern Medicine), and folk remedies.

Bee venom is known to reduce edema of inflammation-induced animals. It is also known that bee venom acts on the synoviocytes of arthritis patients to allow its major ingredient, melittin, to inactivate NF-KB, which reduces the amount of nitric oxide (NO) and inhibits cyclooxygenase-2 (COX-2) to induce anti-inflammatory effects. Physiological research results confirming the therapeutic effects of isolated and purified ingredients of bee venom on diseases, such as dementia and cancer, and research results regarding the analysis of ingredients of dry bee venom have already been reported in the literature. In Oriental medicine, bee venom has long been used as a therapeutic agent for inflammatory diseases and is currently being investigated for its anti-inflammatory and anticancer effects. Many Korean governmental organizations, including the Rural Development Administration, have made research efforts to develop therapeutic agents using bee venom for arthritis or inflammation in animals.

The present inventors have found that the use of a mixture of a lipid-soluble fraction and a water-soluble fraction extracted from bee venom in a predetermined ratio is effective for anti-inflammation and anti-aging, such as reduction of skin wrinkles, and finally arrived at the present invention.

### Detailed Description of the Invention

### Problems to be Solved by the Invention

One object of the present invention is to provide a cosmetic composition containing a predetermined proportion of a lipid-soluble fraction of bee venom that has excellent anti-aging effects, such as reduction of skin wrinkles and prevention of hair loss.

A further object of the present invention is to provide methods for preparing the anti-aging cosmetic composition containing a predetermined proportion of a lipid-soluble fraction of bee venom.

### Means for Solving the Problems

According to one aspect of the present invention, there is provided a cosmetic composition including a lipid-soluble fraction and a water-soluble fraction obtained by fractionation of bee venom with a lipid-soluble solvent and a water-soluble solvent, respectively, in a volume ratio of 1:0.01-0.5. The volume ratio of the lipid-soluble fraction to the water-soluble fraction is preferably adjusted to 1:0.01-0.2, more preferably 1:0.1-0.2. Within this range, a strong protective activity of the cosmetic composition against oxidative damage is provided.

The lipid-soluble solvent is selected from the group consisting of ethyl acetate, chloroform, ether, butanol, and mixtures thereof. The water-soluble solvent is selected from the group consisting of water, ethyl alcohol, methyl alcohol, and mixtures thereof. The cosmetic composition includes at least one non-protein ingredient selected from cytidine, adenosine, chrysin, 11-eicosenol, and pinocembrin. The cosmetic composition includes at least one protein ingredient selected from melittin, phospholipase A-2, and MCD peptide. The water-soluble fraction and the lipid-soluble fraction of bee venom as active ingredients are preferably included in a total amount of 0.0001 to 2% by weight, based on the total weight of the cosmetic composition. Preferably, the cosmetic composition is prepared into a cosmetic for the reduction of skin wrinkles.

According to a further aspect of the present invention, there is provided a method for preparing a cosmetic composition, including: primarily fractionating bee venom with a water-soluble solvent and a lipid-soluble solvent (S10); secondarily fractionating the water-soluble layer obtained after the primary fractionation with a lipid-soluble solvent (S20); tertiarily fractionating the water-soluble layer obtained after the secondary fractionation with a lipid-soluble solvent (S30); removing insoluble matter from the water-soluble layer obtained after the tertiary fractionation by filtration to prepare a water-soluble fraction (S40); combining the lipid-soluble layer obtained after the primary fractionation, the lipid-soluble layer obtained after the secondary fractionation, and the lipid-soluble layer obtained after the tertiary fractionation and removing insoluble matter from the combined lipid-soluble layers by filtration to prepare a lipid-soluble fraction (S50); mixing the water-soluble fraction with the lipid-soluble fraction in a volume ratio of 0.01-0.5:1 (S60); and concentrating and drying the mixture (S70).

According to another aspect of the present invention, there is provided a method for preparing a cosmetic composition, including: extracting bee venom with a water-soluble solvent (S11); centrifuging the bee venom extract, separating the water-soluble layer, and removing insoluble matter from the water-soluble layer by filtration to prepare a water-soluble fraction (S21); drying the centrifuged residue to remove the water-soluble solvent (S31); extracting the dried residue with a lipid-soluble solvent (S41); removing insoluble matter from the extract by filtration to prepare a lipid-soluble fraction (S51); mixing the water-soluble fraction with the lipid-soluble fraction in a volume ratio of 0.01-0.5:1 (S61); and concentrating and drying the mixture (S71).

The lipid-soluble solvent is selected from the group consisting of ethyl acetate, chloroform, ether, butanol, and mixtures thereof. The water-soluble solvent is selected from the group consisting of water, ethyl alcohol, methyl alcohol, and mixtures thereof.

### Effects of the Invention

According to the methods of the present invention, a bee venom extract is prepared in which a lipid-soluble fraction and a water-soluble fraction of bee venom are mixed in a predetermined ratio. The bee venom extract has excellent anti-aging effects, such as reduction of skin wrinkles.

### Brief Description of the Drawings

Fig. 1 is a flowchart showing a method for preparing an extract according to one embodiment of the present invention.
Fig. 2 is a flowchart showing a method for preparing an extract according to a further embodiment of the present invention.
Fig. 3 shows test results for the cytotoxicity of a sample prepared in Example 1.
Fig. 4 shows inhibitory effects of samples prepared in Examples 1-5 on MMP-1 expression.
Fig. 5 shows the effects of samples prepared in Examples 1-5 on procollagen-I synthesis.
Fig. 6 shows the protective activities of samples prepared in Examples 3-5 against oxidative damage.

### Best Mode for Carrying out the Invention

The present invention will now be described in detail.

Bee venom is composed of water-soluble, lipid-soluble, and insoluble ingredients. The use of bee venom has been limited to its water-soluble fractions, mainly protein or peptide fractions containing melittin as a major ingredient.

The present invention provides an anti-aging cosmetic composition using a lipid-soluble fraction of bee venom as a raw material. In the cosmetic composition of the present invention, the use of non-protein and non-peptide fractions of bee venom is appropriately re-adjusted with peptide fractions, etc.

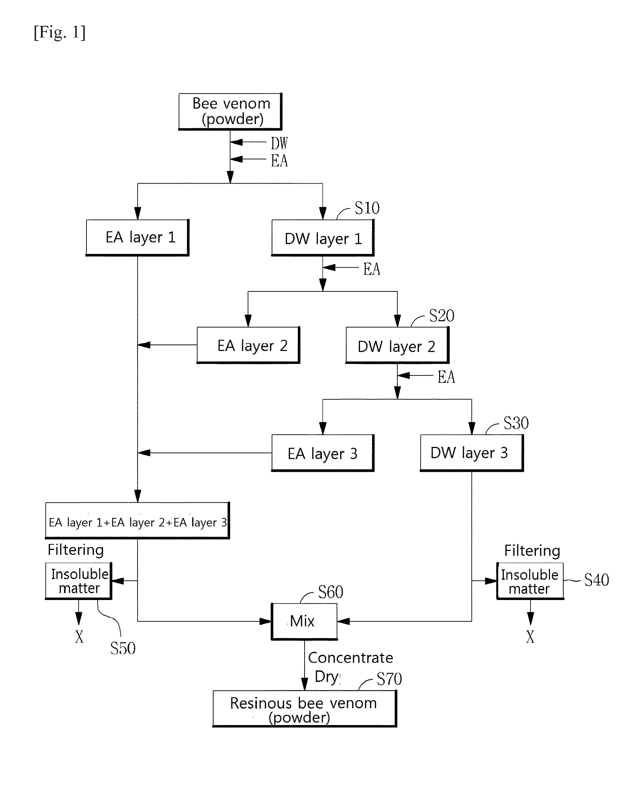

According to one embodiment, the cosmetic composition of the present invention is prepared by a method including: primarily fractionating bee venom with a water-soluble solvent and a lipid-soluble solvent (S10); secondarily fractionating the water-soluble layer obtained after the primary fractionation with a lipid-soluble solvent (S20); tertiarily fractionating the water-soluble layer obtained after the secondary fractionation with a lipid-soluble solvent (S30); removing insoluble matter from the water-soluble layer obtained after the tertiary fractionation by filtration to prepare a water-soluble fraction (S40); combining the lipid-soluble layer obtained after the primary fractionation, the lipid-soluble layer obtained after the secondary fractionation, and the lipid-soluble layer obtained after the tertiary fractionation and removing insoluble matter from the combined lipid-soluble layers by filtration to prepare a lipid-soluble fraction (S50); mixing the water-soluble fraction with the lipid-soluble fraction in a volume ratio of 0.01-0.5:1 (S60); and concentrating and drying the mixture (S70). This method is shown in Fig. 1. In the Examples section that follows, distilled water was used as the water-soluble solvent and ethyl acetate (EA) was used as the lipid-soluble solvent, as shown in Fig. 1.

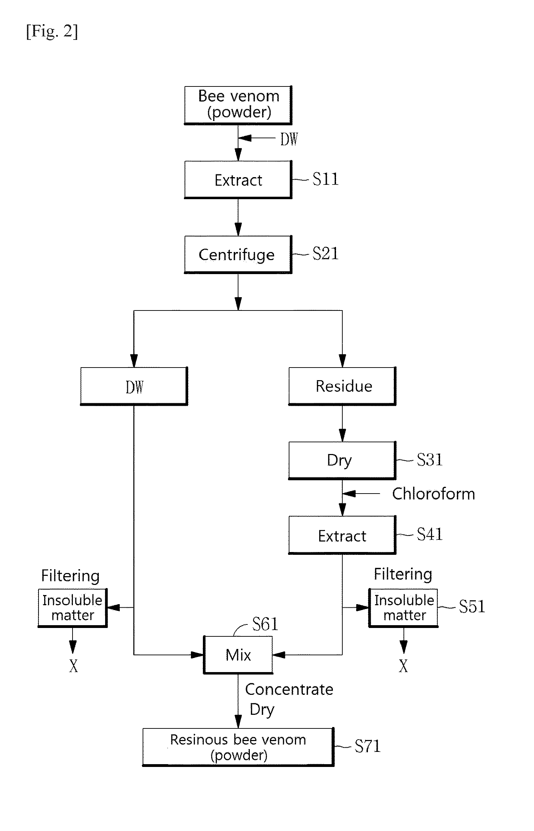

According to another embodiment, the cosmetic composition of the present invention is prepared by a method including: extracting bee venom with a water-soluble solvent (S11); centrifuging the bee venom extract, separating the water-soluble layer, and removing insoluble matter from the water-soluble layer by filtration to prepare a water-soluble fraction (S21); drying the centrifuged residue to remove the water-soluble solvent (S31); extracting the dried residue with a lipid-soluble solvent (S41); removing insoluble matter from the extract by filtration to prepare a lipid-soluble fraction (S51); mixing the water-soluble fraction with the lipid-soluble fraction in a volume ratio of 0.01-0.5:1 (S61); and concentrating and drying the mixture (S71). This method is shown in Fig. 2. In the Examples section that follows, chloroform was used as the lipid-soluble solvent and distilled water was used as the water-soluble solvent. Specifically, the cosmetic composition may be prepared by the following procedure. 1.0 g of bee venom is extracted with 100 ml of distilled water in a beaker. The extract is centrifuged at 3,000 rpm for 10 minutes. The water-soluble layer (DW layer) is separated, filtered to remove insoluble matter, and stored. The centrifuged residue is dried to remove a slight amount of remaining distilled water. The dried residue is extracted with 200 ml of chloroform and filtered to remove insoluble matter. The distilled water layer is mixed with the chloroform layer. Then, the mixture is concentrated and dried.

The ingredient profiles of the fractions obtained by the methods were investigated. The results showed that the extracts contain cytidine, adenosine, chrysin, 11-eicosenol, and pinocembrin as non-protein ingredients and melittin, phospholipase A-2, apamin, and MCD peptide as protein ingredients.

The water-soluble fraction and the lipid-soluble fraction were mixed in different ratios. As a result, particularly when the volume ratio of the lipid-soluble fraction to the water-soluble fraction was 1:0.01-0.2, more specifically 1:0.1-0.2, a strong recovery activity was obtained against oxidative damage.

The water-soluble fraction and the lipid-soluble fraction of bee venom as active ingredients are preferably included in a total amount of 0.001 to 20% by weight, based on the total weight of the cosmetic composition.

The bee venom extract may be prepared into an anti-aging cosmetic. In this case, the anti-aging cosmetic may be prepared using a diluent or an excipient. The diluent or excipient may be any of those commonly used in the art, for example, a filler, an extender, a binder, a wetting agent, a disintegrating agent or a surfactant. The diluent may be water or liquid paraffin. The cosmetic may also include a vehicle, a sterile aqueous solution, a non-aqueous solvent, a suspending agent, an emulsifier or a freeze-drying agent. As the non-aqueous solvent or suspending agent, there may be used, for example, propylene glycol, polyethylene glycol, an ester, such as ethyl oleate, and a vegetable oil, such as olive oil.

### [EXAMPLES]

### Example 1: Preparation of extract including lipid-soluble fraction

2.5 g of crude bee venom (CBV) was dissolved in 100 ml of distilled water (DW) in a beaker. The bee venom solution was transferred to a separatory funnel and 150 ml of distilled water was added thereto while washing off the bee venom solution on the inner wall of the beaker. 250 ml of ethyl acetate (EA) was added to and mixed with the solution with shaking. The resulting mixture solution was allowed to stand in the separatory funnel and the stopcock was opened to vent the pressure in the funnel. The EA layer and the distilled water layer were separated from each other due to their different properties. The lower distilled water layer was drained out and collected in a beaker. The upper EA layer was separately collected. The distilled water layer was transferred to a separatory funnel and 250 ml of EA was added thereto. After sufficiently mixing, the mixture was allowed to stand for phase separation. The upper EA layer was separately collected. The lower distilled water layer was transferred to a separatory funnel and 250 ml of EA was added thereto. After sufficient mixing, the mixture was allowed to stand for phase separation. The separated EA layers were combined and insoluble matter were removed therefrom to prepare a lipid-soluble fraction. Insoluble matter was removed from the separated distilled water layer to prepare a water-soluble fraction. The water-soluble fraction and the lipid-soluble fraction were mixed in a volume ratio of 0.5:1. The mixture was concentrated and dried.

The sample was dissolved in a solvent before use.

### Example 2: Preparation of extract including lipid-soluble fraction

An extract was prepared in the same manner as in Example 1, except that the water-soluble fraction and the lipid-soluble fraction were mixed in a volume ratio of 0.3:1.

### Example 3: Preparation of extract including lipid-soluble fraction

An extract was prepared in the same manner as in Example 1, except that the water-soluble fraction and the lipid-soluble fraction were mixed in a volume ratio of 0.2:1.

### Example 4: Preparation of extract including lipid-soluble fraction

An extract was prepared in the same manner as in Example 1, except that the water-soluble fraction and the lipid-soluble fraction were mixed in a volume ratio of 0.1:1.

### Example 5: Preparation of extract including lipid-soluble fraction

An extract was prepared in the same manner as in Example 1, except that the water-soluble fraction and the lipid-soluble fraction were mixed in a volume ratio of 0.01:1.

The extracts prepared in Examples 1-5 as samples were tested for anti-aging efficacy.

### Test Example 1: Cytotoxicity test

Cell proliferation was measured using an EZ-cytox enhanced cell viability assay kit (Daeil Lab, Korea). Experiments were conducted according to the manufacturer's instructions.

Normal human dermal fibroblasts (NHDF) were plated in each well of a 48-well plate at a density of 5000 cells/well and cultured in DMEM (low glucose) supplemented with 1% FBS at 37 °C for 1 day. The culture was treated with different concentrations of the extract of Example 1 (lipid and water soluble bee venom (LWBV)). After incubation for 24 and 48 h, CCK assay was performed. An assay reagent was added so as to account for 10% of the medium. After incubation was performed under the same conditions for 2 h, the absorbance at 450 nm was measured with a microplate reader (Bio-Rad, USA).

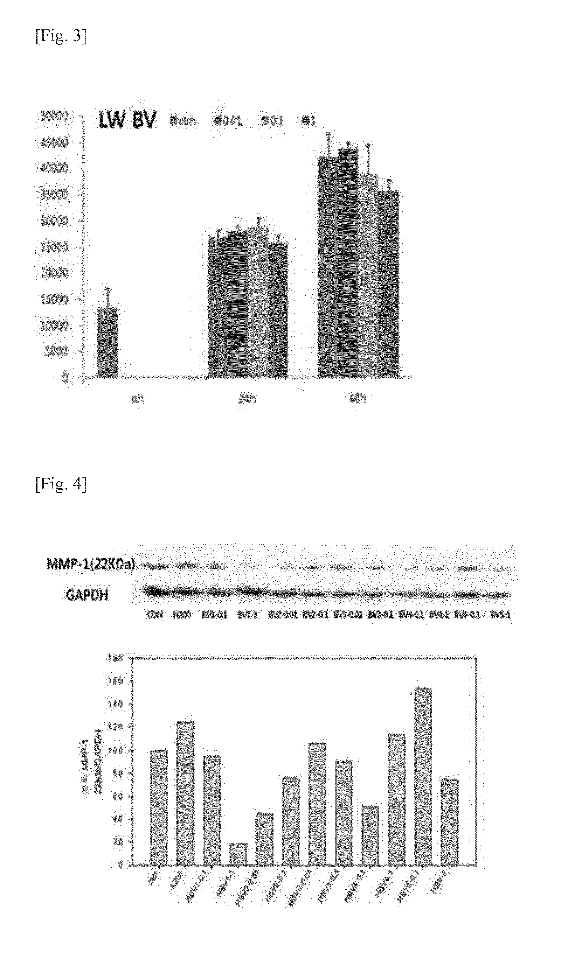

The results are shown in Fig. 3. As can be seen from Fig. 3, LWBV caused no toxicity to the fibroblasts at all concentrations.

### Test Example 2: Inhibitory effects on MMP-1 expression

Human dermal fibroblasts were pretreated with different concentrations of the bee venom extracts prepared in Examples 1-5 and were then exposed to 200 µM hydrogen peroxide, which is a concentration at which apoptosis is not induced. After 24 h-incubation, mRNA was isolated and the gene expression level of MMP-1 was determined by RT-PCR.

The results are shown in Fig. 4. Hydrogen peroxide (H200) increased the MMP-1 expression of fibroblasts and the bee venom extracts reduced the MMP-1 expression as a whole. Particularly, the bee venom extract of Example 1 (BV1) showed a superior inhibitory effect on MMP-1 expression. In Fig. 4, HBV1-1 refers to the sample having undergone exposure to hydrogen peroxide after treatment with 1 µg/ml of the bee venom extract of Example 1. Likewise, HBV4-0.1 refers to the sample having undergone exposure to hydrogen peroxide after treatment with 0.1 µg/ml of the bee venom extract of Example 4.

### Test Example 3: Effects on procollagen-I synthesis

Human dermal fibroblasts were pretreated with different concentrations of the bee venom extracts prepared in Examples 1-5 and were then exposed to 200 µM hydrogen peroxide. After 24-h incubation, proteins were isolated from the cells and the amount of procollagen-I synthesized was determined by Western blotting.

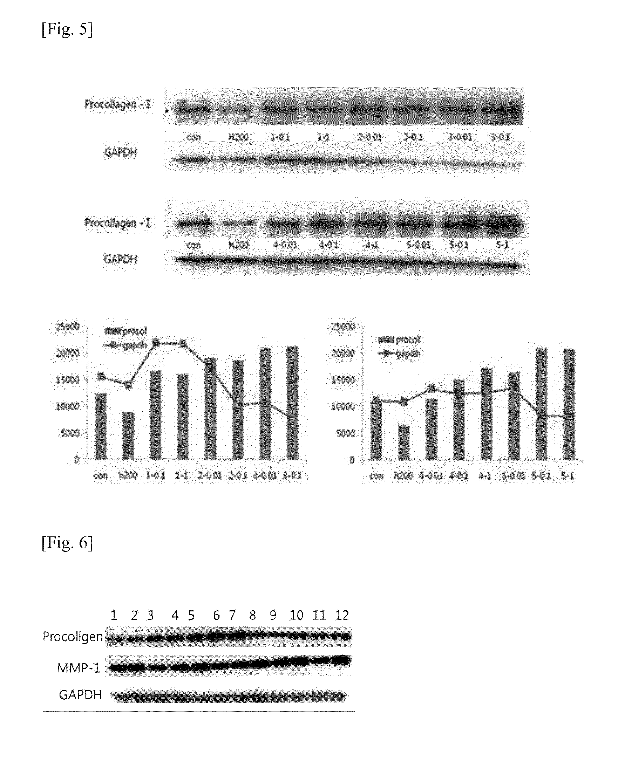

The results are shown in Fig. 5. Hydrogen peroxide inhibited the synthesis of collagen by the fibroblasts and all bee venom extracts of Examples 1-5 increased the synthesis of collagen.

### Test Example 4: Protective effects against oxidative damage

The following experiments were conducted to confirm the protective activity of the bee venom fractions against oxidative stress. Human dermal fibroblasts were pretreated with different concentrations of the bee venom extracts prepared in Examples 3-5 and were then exposed to 400 µM hydrogen peroxide. After 24-h incubation, the expression profiles of MMP-1 and procollagen by oxidative damage were analyzed to investigate the cell protective functions of the bee venom fractions against oxidative damage and the ability of the bee venom fractions to control aging.

The results are shown in Fig. 6. The numbers in Fig. 6 indicate the following conditions.

1: N.C, 2: H₂O₂ (400 µM), 3: Example 5 (3 µg/ml), 4: Example 3 (2 µg/ml), 5: Example 3 (3 µg/ml), 6: Example 4 (2 µg/ml), 7: Example 4 (3 µg/ml), 8: Example 5 (3 µg/ml) + H₂O₂ (400 µM), 9: Example 3 (2 µg/ml) + H₂O₂ (400 µM), 10: Example 3 (3 µg/ml) + H₂O₂ (400 µM), 11: Example 4 (2 µg/ml) + H₂O₂ (400 µM), 12: Example 4 (3 µg/ml) + H₂O₂ (400 µM)

As confirmed from Fig. 6, the extracts of Examples 3 and 5 were effective in recovering the amount of procollagen, which had been reduced by hydrogen peroxide treatment. The extract of Example 4 was very effective in recovering the expressions of both procollagen and MMP-1 to the levels before oxidative damage. These results demonstrate very good cell protective activity of the extracts of Examples 3-5 against oxidative damage.

As confirmed in Test Examples 1-4, the treatment with the compositions of Examples 1-5 increased the turnover of dermal tissues, resulting in enhanced collagen synthesis and inhibited collagen degradation. These results reveal that when exposed to stress forming free radicals, the bee venom extracts of Examples 1-5 have protective effects to regulate the metabolism of the extracellular matrix (ECM) and rejuvenation effects to induce the synthesis of new collagen, thus being suitable for use in anti-aging cosmetics.

### Industrial Applicability

The bee venom extract prepared by the methods of the present invention, in which a lipid-soluble fraction and a water-soluble fraction of bee venom are mixed in a predetermined ratio, has excellent anti-aging effects, such as reduction of skin wrinkles, due to its good protective activity against oxidative damage. Therefore, the bee venom extract of the present invention can be useful in the cosmetics industry.

## Claims

1. A cosmetic composition comprising a lipid-soluble fraction and a water-soluble fraction obtained by fractionation of bee venom with a lipid-soluble solvent and a water-soluble solvent, respectively, in a volume ratio of 1:0.01-0.5.

2. The cosmetic composition according to claim 1, wherein the lipid-soluble fraction and the water-soluble fraction are in a volume ratio of 1:0.01-0.2.

3. The cosmetic composition according to claim 2, wherein the volume ratio of the lipid-soluble fraction to the water-soluble fraction is adjusted to 1:0.1-0.2 to protect the skin against oxidative damage.

4. The cosmetic composition according to claim 1, wherein the lipid-soluble solvent is selected from the group consisting of ethyl acetate, chloroform, ether, butanol, and mixtures thereof.

5. The cosmetic composition according to claim 1, wherein the water-soluble solvent is selected from the group consisting of water, ethyl alcohol, methyl alcohol, and mixtures thereof.

6. The cosmetic composition according to claim 1, wherein the cosmetic composition comprises at least one non-protein ingredient selected from cytidine, adenosine, chrysin, 11-eicosenol, and pinocembrin.

7. The cosmetic composition according to claim 1, wherein the cosmetic composition comprises at least one protein ingredient selected from melittin, phospholipase A-2, and MCD peptide.

8. The cosmetic composition according to claim 1, wherein the cosmetic composition is prepared into a cosmetic for the reduction of skin wrinkles.

9. A method for preparing a cosmetic composition, comprising: primarily fractionating bee venom with a water-soluble solvent and a lipid-soluble solvent (S10); secondarily fractionating the water-soluble layer obtained after the primary fractionation with a lipid-soluble solvent (S20); tertiarily fractionating the water-soluble layer obtained after the secondary fractionation with a lipid-soluble solvent (S30); removing insoluble matter from the water-soluble layer obtained after the tertiary fractionation by filtration to prepare a water-soluble fraction (S40); combining the lipid-soluble layer obtained after the primary fractionation, the lipid-soluble layer obtained after the secondary fractionation, and the lipid-soluble layer obtained after the tertiary fractionation and removing insoluble matter from the combined lipid-soluble layers by filtration to prepare a lipid-soluble fraction (S50); mixing the water-soluble fraction with the lipid-soluble fraction in a volume ratio of 0.01-0.5:1 (S60); and concentrating and drying the mixture (S70).

10. A method for preparing a cosmetic composition, comprising: extracting bee venom with a water-soluble solvent (S11); centrifuging the bee venom extract, separating the water-soluble layer, and removing insoluble matter from the water-soluble layer by filtration to prepare a water-soluble fraction (S21); drying the centrifuged residue to remove the water-soluble solvent (S31); extracting the dried residue with a lipid-soluble solvent (S41); removing insoluble matter from the extract by filtration to prepare a lipid-soluble fraction (S51); mixing the water-soluble fraction with the lipid-soluble fraction in a volume ratio of 0.01-0.5:1 (S61); and concentrating and drying the mixture (S71).

11. The method according to claim 9 or 10, wherein the lipid-soluble solvent is selected from the group consisting of ethyl acetate, chloroform, ether, butanol, and mixtures thereof and the water-soluble solvent is selected from the group consisting of water, ethyl alcohol, methyl alcohol, and mixtures thereof.
